(19) Europäisches Patentamt
European Patent Office
Office européen des brevets

(11) Publication number: 0 040 991
B1

(12) **EUROPEAN PATENT SPECIFICATION**

(45) Date of publication of patent specification: 18.07.84

(51) Int. Cl.³: C 07 C 121/75, A 01 N 37/38, C 07 B 19/00

(21) Application number: 81302345.4

(22) Date of filing: 27.05.81

# (54) Preparation of insecticidal mixture of stereoisomers of alpha-cyano-3-phenoxybenzyl isovalerates.

(30) Priority: 28.05.80 JP 71940/80

(43) Date of publication of application:
02.12.81 Bulletin 81/48

(45) Publication of the grant of the patent:
18.07.84 Bulletin 84/29

(84) Designated Contracting States:
CH DE FR GB IT LI NL

(56) References cited:
EP - A - 0 002 289
GB - A - 2 001 964
GB - A - 2 013 206
GB - A - 2 014 137
US - A - 4 213 916
US - A - 4 238 406

(73) Proprietor: SUMITOMO CHEMICAL COMPANY, LIMITED
15 Kitahama 5-chome Higashi-ku
Osaka-shi Osaka-fu (JP)

(72) Inventor: Suzuki, Yukio Sumito Chemical Apt. No. 2-241
No. 10, Sone Higashi-machi 2-chome Toyonaka-shi
Osaka (JP)
Inventor: Hayashi, Masahiro
No. 7-307, No. 11, Sone Higashi-machi 2-chome
Toyonaka-shi Osaka (JP)
Inventor: Takuma, Kenzi
No. 6-3, Nishi mami 2-chome Kashiba-cho
Kita Katsuragi-gun Nara (JP)

(74) Representative: Diamond, Bryan Clive et al,
Gee & Co. Chancery House Chancery Lane
London WC2A 1QU (GB)

EP 0 040 991 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

## Description

This invention relates to a method of preparing a mixture of stereoisomers of α-cyano-3-phenoxybenzyl isovalerate ester derivatives of the general formula (I):

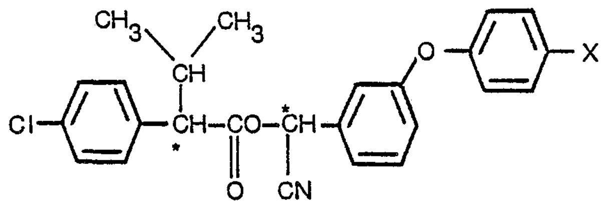

(I)

wherein X is a hydrogen atom or a fluorine atom, and each * indicates an asymmetric carbon atom, which mixture is rich in an enantiomer pair of a compound of the formula (I) having an (S)-configuration on both the acid and alcohol moieties and its enantiomer.

α-Cyano-3-phenoxybenzyl 2-(4-chlorophenyl)isovalerate (hereinafter referred to as "fenvalerate") and α-cyano-3-(4-fluorophenoxy)-benzyl 2-(4-chlorophenyl)isovalerate (referred to as "p-F fenvalerate"), the two compounds of the above formula (I), are useful as insecticides and/or acaricides, as disclosed in, for example, U.S. Patent 4,062,968 and British Patent 1,549,462. These esters are of the above formula (I) and have one asymmetric carbon atom on each of the acid and alcohol moieties.

These optical isomers are hereunder referred to as shown in Table 1 below.

TABLE 1
Abbreviations for Optical Isomers

| | Acid Moiety | | |
|---|---|---|---|
| Alcohol Moiety | (S)-Configuration | Racemic | (R)-Configuration |
| (S)-Configuration | Aα-Isomer | α-Isomer | Bα-Isomer |
| Racemic | A-Isomer | Racemate | B-Isomer |
| (R)-Configuration | Aβ-Isomer | β-Isomer | Bβ-Isomer |

Combinations of the Aα-isomer and the Bβ-isomer and of the Aβ-isomer and the Bα-isomer are each in an enantiomer pair relationship. The enantiomer pair of the Aα-isomer and Bβ-isomer is referred to herein as a Y-isomer, and the enantiomer pair of the Aβ-isomer and the Bα-isomer as an X-isomer. An ester provided by the conventional method is a mixture comprising substantially equal amounts of four isomers, i.e. a "racemate".

According to the disclosure of U.S. Patent 4,238,406, the insecticidal and/or acaricidal efficacy of the Y-isomer of fenvalerate is about twice as high as that of racemic fenvalerate (the racemate wherein the ratio of X-isomer to Y-isomer is about 1:1; hereinafter unless otherwise stated, "racemic fenvalerate" refers to fenvalerate of this composition), and the Y-isomer or the ester rich in the Y-isomer can be obtained by crystallizing the Y-isomer out of racemic fenvalerate in the presence or absence of a base. It is well known that the Y-isomer of fenvalerate forms crystals (m.p. 40°C). Further, GB 2,001,964 A discloses that both the Aα-isomer and the Bβ-isomer of fenvalerate form crystals (m.p. 57.9°C and 59.6°C respectively). But nothing has hitherto been known about crystallization of the Aβ-isomer, Bα-isomer and X-isomer (the mixture thereof) of fenvalerate.

Likewise, with respect to p-F fenvalerate, it is disclosed in GB 2,041,366 A that the insecticidal and/or acaricidal efficacy of the Y-isomer is about twice as high as that of racemic p-F fenvalerate (the racemate, the meaning of the term "racemic" is the same as for fenvalerate), and the Y-isomer or the ester rich in the Y-isomer can be obtained by crystallizing the Y-isomer out of racemic p-F fenvalerate; and that the Aα-isomer of p-F fenvalerate forms crystals. But nothing has hitherto been known about crystallization of the Aβ-isomer, Bα-isomer and X-isomer (mixture thereof) of p-F fenvalerate.

Specification GB 2,014,137 A disclosed an optically active α-cyano-3-phenoxybenzyl 2-(4-chlorophenyl)isovalerate and an insecticidal composition thereof;
the method for obtaining the Aα-isomer is by crystallizing the Aα-isomer from a solution of the A-isomer. The "(S)-α-cyano-3-phenoxybenzyl "D"-2-isopropyl-2-*p*-chlorophenylacetate" referred to in No. 2,014,137 corresponds to (S)-α-cyano-3-phenoxybenzyl (S)-2-(4-chlorophenyl)isovalerate, i.e. the Aα-isomer of fenvalerate, referred to in this application.

E.P. 0002289 discloses a method for treating (R)-α-cyano-3-phenoxybenzyl (S)-2-(4-chlorophenyl)isovalerate with a base to convert it into (S)-α-cyano-3-phenoxybenzyl (S)-2-(4-chlorophenyl)isovalerate, i.e. an epimerization method. The "(+)-R-*alpha*-cyano-3-phenoxybenzyl (+)-S-*alpha*-isopropyl-4-chlorophenylacetate" to which 0002289 refers corresponds to (R)-α-cyano-3-phenoxybenzyl (S)-2-(4-chlorophenyl)-isovalerate, i.e. the Aβ-isomer of fenvalerate, referred to in this application. Further, "(—) - S - *alpha* - cyano - 3 - phenoxybenzyl (+) - S - alpha - isopropyl - 4 - chlorophenylacetate" corresponds to (S)-α-cyano-3-phenoxybenzyl (S)-2-(4-chlorophenyl)isovalkerate, i.e. the Aα-isomer of fenvalerate.

Although these prior specifications disclose that the Aα-isomer of fenvalerate is crystalline, they do not suggest that the X-isomer of fenvalerate (i.e. an enantiomer pair of Aβ-isomer and Bα-isomer) to which this invention is the subject, is crystalline. In addition, in these prior methods, the starting materials are isomers that are optically active in the acid moiety thereof, i.e. those obtainable from optical resolution operation, whereas in the method of the present invention, the racemate which can be obtained by conventional methods is used as the starting material to provide the Y-isomer having a higher insecticidal activity.

We have now found that the X-isomer of fenvalerate crystallizes (m.p. 63° to 66°C), and that even the racemic fenvalerate, hitherto only known as a viscous liquid, can crystallize (m.p. 37.0° to 53.6°C), and that the X-isomer of p-F fenvalerate also crystallizes (m.p. 76.5° to 78.0°C).

It has also been found that by crystallizing the X-isomer or a mixture of the ester rich in the X-isomer from the racemate and removing the resulting X crystals, the ester rich in the Y-isomer can be obtained from the mother liquor.

The invention provides a method of preparing the aforesaid Y-isomer of the ester of formula (I), wherein from a mixture of the aforesaid X-isomer and the Y-isomer of the ester crystals are removed which are rich in the X-isomer, whereby the Y-rich isomer mixture is obtained from the mother liquor; preferably X-rich isomer is removed by crystallizing it out of a solution of the ester of the formula (I) which is a mixture of the X-isomer and the Y-isomer in the presence of crystals which consist entirely or principally of X-isomer.

In the methods described in U.S. Patent 4,238,406 and GB 2,041,366A, the crystallization of the Y-isomer proceeds extremely slowly, so a long period of time is taken for completion of the crystallization, even by adding large amounts of seed crystals of Y-isomer. In the method of the present invention, however, the crystallization of the X-rich isomer mixture proceeds rapidly so that it is completed in approximately several hours. This method is therefore commercially more advantageous.

The mixture rich in X-isomer separated from the racemic ester as also described in U.S. Patent 4,238,406 and GB 2,041,366A can be returned to the racemate (epimerization), an approximately 1:1 mixture of the X-isomer and the Y-isomer, by bringing it into contact with a basic catalyst. Consequently, the Y-rich isomer mixture can be obtained approximately quantitatively from the racemic ester by repeated cycles of the crystallization and the epimerization.

The ratio of the X-isomer to the Y-isomer present in the starting ester is preferably within the range of 35:65 to 65:35. The ester starting material may be of liquid or solid form. It is better that both the acid and alcohol moieties of the starting ester are in a racemic form, but they need not always be a complete racemate.

The crystallization of the X-isomer or X-rich isomer mixture is preferably carried out in a solvent, because the starting ester in liquid form is viscous at the crystallization temperature of the X-isomer. Suitable examples of the solvent which can be used include alcohols such as lower alcohols having 1 to 4 carbon atoms (e.g., methanol, ethanol, *n*-propanol, isopropanol or *n*-butanol), aliphatic hydrocarbons (e.g., pentane, heptane, hexane, petroleum ether or ligroin), alicyclic hydrocarbons (e.g., cyclohexane or methylcyclohexane), and mixtures containing at least one of these solvents. Suitable examples of a mixed solvent are mixtures of at least one of the alcohols, aliphatic hydrocarbons, alicyclic hydrocarbons and mixtures of two or more of these solvents, with aromatic hydrocarbons (e.g., toluene, benzene or xylene). The amount of the solvent is generally 0.5 to 10 times the weight of the ester used as the starting material.

The epimerization of this ester, which may occur when the alcohol or a mixed solvent containing the same is used as a solvent, can be avoided by the addition of an acidic substance (e.g., acetic acid, hydrogen chloride or sulfuric acid).

The crystallization can be carried out in the presence of crystalline X-isomer or an X-rich mixture. Generally, it is carried out by dissolving the starting ester in a solvent and adding the aforesaid crystal as a seed crystal at a temperature at which the crystallization takes place. But the addition of the seed crystal is not necessary when the crystal is already contained in the crystallization system without being completely dissolved or melted. The crystallization out of the X-rich isomer mixture can be carried out batchwise, continuously or semi-continuously. When the crystallization is carried out continuously or semi-continuously, seeding with crystals may be effected only at the initiation of the crystallization.

The minimum amount of X-isomer in the X-rich crystals removed may suitably be 59%, and the minimum amount of Y-isomer in the mother liquor may be 59%.

The method may be carried out in the following manner: the solution of the racemic ester is passed through an apparatus where the seed crystals of the X-isomer or the X-rich mixture are held and which is kept at a temperature at which the crystallization and precipitation of the X enantiomer takes place in the apparatus, to leave a solution rich in the Y enantiomer.

When the starting ester is of substantially solid form, mere washing or partial dissolving of the ester with a solvent enables the Y-rich ester to be obtained from the washings.

The temperature at which the crystallization is carried out is preferably 40°C or less, more preferably −30°C to 30°C. The precipitated X-rich ester is then separated from the mother liquid by a usual method used to separate solid from liquid, such as filtration, decantation or centrifugation. The Y-rich ester is thus recovered from the mother liquor.

The Y-isomer can be obtained from the Y-rich ester thus-obtained, if necessary, by recrystallization or other means.

The method of this invention is hereunder described in greater detail by reference to the following reference examples and examples.

In the following reference examples and examples, the ratio of X-isomer to Y-isomer was analyzed by gas chromatography. The analytical conditions were as follows:

Tube: 3 mm diameter × 3.0 m, packed with Chromosorb absorbent AW—DMCS coated with 10% silicon DC QF-1
Analytical Temperature: 245°C
Injection Temperature: 250°C
Nitrogen Pressure: 2.0 kg/cm$^2$

Concentrations of mother liquor were performed by evaporation in vacuo.

In the following reference examples and examples, unless otherwise stated, fenvalerate, p-F fenvalcrate, fenvalerate X, p-F fenvalerate X, fenvalerate Y and p-F fenvalerate Y mean racemates as regards both the acid and alcohol moieties, and the ratios of X-isomer to Y-isomer in fenvalerate and p-F fenvalerate were 50:50, respectively, unless otherwise stated.

### Reference Example 1

810 mg of fenvalerate was dissolved in hexane, and the solution was adsorbed on a silica gel column (Lobar Column Lichroprep Si 60, Size B, a Trade Mark of Merk Co.) and then eluted with a hexane/ethyl acetate (100/1 by volume) mixed solvent. Fractions, in which the Y-isomer was hardly detectable by gas chromatography, were combined and concentrated to obtain 212 mg of the X-isomer. This X-isomer was dissolved in 1 cc of heptane, and the solution was allowed to stand, in a tightly sealed condition, for a week in a refrigerator (about 0°C) to provide crystals which were then vacuum-dried to give X-isomer crystals (m.p. 63°C—66.0°C).

### Reference Example 2

700 mg of p-F fenvalerate was dissolved in hexane, and the solution was adsorbed on a silica gel column (Lobar Column Lichroprep Si 60, Size B) and eluted with a hexane/ethyl acetate (80:1 by volume) mixed solvent. Fractions of X-isomer which were eluted as a first peak during the gas chromatography were combined and concentrated to obtain 190 mg of the X-isomer. To this X-isomer of p-F fenvalerate were added the crystals of X-isomer of fenvalerate obtained in Reference Example 1, and the X-isomer of p-F fenvalerate was solidified (m.p. 76.5—78°C).

### Example 1

50 g of fenvalerate was dissolved in 250 g of heptane, and after adding thereto as a seed crystal 1 mg of X-isomer crystals of fenvalerate at room temperature (20—23°C), the solution was stirred for 5 hours as it was. The precipitated crystals were filtered to give 14.80 g of an X-rich (X-isomer/Y-isomer = 70.3/29.7) ester. The mother liquor was concentrated to give a Y-rich ester (X-isomer/Y-isomer = 41.0/59.0).

### Example 2

100 g of fenvalerate (X-isomer/Y-isomer = 53.2/46.8) was dissolved in 200 g of methanol, and after adding thereto as a seed crystal 1 mg of X-isomer crystals of fenvalerate at room temperature (20°—23°C), the solution was stirred for 5 hours as it was. The precipitated crystals were removed by filtration, and the mother liquor was concentrated to give 65.02 g of a Y-rich ester (X-isomer/Y-isomer = 38.7/61.3).

### Example 3

50 g of fenvalerate (X-isomer/Y-isomer = 53.2/46.8) was dissolved in 50 g of methanol, and the solution was cooled to 5° to 7°C. To the solution was added as a seed crystal 1 mg of X-isomer crystals of fenvalerate, and the resulting solution was stirred for 4 hours at the same temperature. The precipitated crystals were removed by filtration, and the mother liquor was concentrated to give 14.78 g of Y-rich ester (X-isomer/Y-isomer = 22.6/77.4).

### Reference Example 3

To 100 g of liquid-form fenvalerate was added 100 g of heptane, followed by stirring at room temperature (20—23°C). One hour after the stirring, seed crystals of X-isomer and Y-isomer of

fenvalerate were added thereto, followed by stirring for 16 hours at the same temperature. A nitrogen stream was passed through the system to evaporate most of the heptane in 5 hours. The remaining semi-solid product was vacuum-dried to obtain solid-form fenvalerate (m.p. 37,0°—53.6°C).

Example 4

To 7 g of solid-form fenvalerate obtained in Reference Example 3 was added 7 g of methanol at room temperature (20°—23°C), followed by stirring at the same temperature. 5.5 hours after the stirring, the crystals were removed by filtration, and the mother liquor was concentrated to give 3.03 g of Y-rich ester (X-isomer/Y-isomer = 34.5/65.5).

Example 5

100 g of fenvalerate (X-isomer/Y-isomer = 53.2/46.8) was dissolved in 200 g of methanol, and the solution was cooled to −18°C. To the solution was added 1 mg of X-rich crystals of fenvalerate obtained in Example 1, and the resulting solution was stirred for 5 hours at the same temperature. The precipitated crystals were removed by filtration, and the mother liquor was concentrated to give 33.58 g of a Y-rich (X-isomer/Y-isomer = 23.6/76.4) ester.

Example 6

100 g of fenvalerate (X-isomer/Y-isomer = 53.2/46.8) was dissolved in 200 g of isopropyl alcohol, and after adding thereto 1 mg of crystal-form fenvalerate, the solution was stirred for 6 hours at room temperature (20—23°C). The precipitated crystals were removed by filtration, and the mother liquor was concentrated to give 52.53 g of a Y-rich ester (X-isomer/Y-isomer = 33.8/66.2).

Example 7

To 7 g of solid-form fenvalerate obtained in Reference Example 3 was added 7 g of methanol and dissolved at 37°C (at that time, no crystals remained visible). The solution was then cooled to 24°C and stirred for 6 hours. The precipitated crystals were removed by filtration, and the mother liquor was concentrated to give 4.87 g of a Y-rich ester (X-isomer/Y-isomer = 40.2/59.8).

Example 8

100 g of fenvalerate (X-isomer/Y-isomer = 53.2/46.8) was dissolved in a mixed solvent comprising 117 g of heptane, 13 g of toluene and 70 g of methanol, and the solution was cooled to 4 to 5°C. To the solution was added 1 mg of X-rich crystals of fenvalerate obtained in Example 1, and the resulting solution was stirred for 5 hours at the same temperature. The precipitated crystals were removed by filtration and the mother liquor was concentrated to give 50.79 g of a Y-rich ester (X-isomer/Y-isomer = 34.4/65.6).

Example 9

The same procedure as in Example 8 was repeated except that a mixed solvent comprising 130 g of heptane and 70 g of methanol was used. Thus, 39.29 g of Y-rich ester (X-isomer/Y-isomer = 28.8/71.2) was obtained.

Example 10

This example is described with reference to the single accompanying drawing.

1 g of X-rich crystals of fenvalerate (X-isomer/Y-isomer = 88.1/11.9) were melted and then solidified on the inner surface of a tubular glass vessel 1 of diameter 36 mm having an inlet 5 and an outlet 6 (inside diameter: 36 mm) up to the level of 130 mm from the bottom. The vessel was placed in a bath 2 kept at −15°C such that the region where the X-rich crystals were attached (the distance A—B was 130 mm) was immersed in the bath 2. Thereafter, a solution of 30 g of fenvalerate (X-isomer/Y-isomer = 53.2/46.8) in 300 g of a methanol solution containing 1.5 g of acetic acid was circulated (at a flow rate of 2.8 ml/min) between the vessel 1 and a flask 3 equipped with a stirrer kept at room temperature (20—23°C) by means of a pump 4 for 2 days. Then, the resulting solution was concentrated to give 16.3 g of a Y-rich ester (X-isomer/Y-isomer = 35.5/64.5).

Example 11

10 g of a X-rich mixture of fenvalerate (X-isomer/Y-isomer = 65.1/34.9) was dissolved in 50 g of methanol. To the solution were added 100 g of soft glass beads having a diameter of 1 mm, and the system was heated under reflux condition for 2 hours. Thereafter, the system was cooled to room temperature (20—23°C), and the liquid portion was separated from the glass beads by decantation. A portion of the solution was sampled and analyzed by gas chromatography; the ratio X-isomer/Y-isomer was 54.5/45.5, which proved that epimerization occurred.

This solution was cooled in a bath kept at −15°C, and after adding thereto a small amount of X-rich crystals (X-isomer/Y-isomer = 65.1/34.9), the resulting solution was allowed to stand for one day at the same temperature. During this procedure, crystals gradually precipitated. The solution was decanted and concentrated to give 2.23 g of a Y-rich ester (X-isomer/Y-isomer = 17.0/83.0). The amount of the crystals thus precipitated was 7.28 g.

Example 12

0.45 g of p-F fenvalerate (X-isomer/Y-isomer = 53.4/46.6) was dissolved in 0.90 g of methanol, and the solution was cooled to 0°C. To the solution was added a small amount of X-isomer crystals of p-F fenvalerate obtained in Reference Example 2, and the resulting solution was allowed to stand at the same temperature for 2 days. Thereafter, the precipitated crystals were collected by filtration to give 0.308 g of an X-rich ester (X-isomer/Y-isomer = 62.3/37.3, m.p. 61.0 to 62.5°C). The mother liquor was concentrated to give 0.141 g of a Y-rich ester (X-isomer/Y-isomer = 32.7/67.3).

## Claims

1. A method of preparing a mixture of stereoisomers of an $\alpha$-cyano-3-phenoxybenzyl isovalerate ester derivative of the general formula:

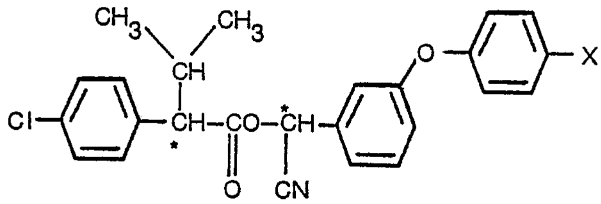

wherein X is a hydrogen atom or a fluorine atom and each * indicates an asymmetric carbon atom, which mixture is rich in a "Y-isomer", which is an enantiomer pair of a compound of said formula having an (S)-configuration on both the acid and alcohol moieties and an enantiomer thereof having an (R)-configuration on both the acid and alcohol moieties, which method comprises removing crystals of a compound of said formula rich in an "X-isomer", which is an enantiomer pair of a a compound of said formula having an (S)-configuration on the acid moiety and an (R)-configuration on the alcohol moiety and an enantiomer thereof having an (R)-configuration on the acid moiety and an (S)-configuration on the alcohol moiety, from an ester of said formula which is a mixture of said X-isomer and said Y-isomer, to obtain an ester rich in said Y-isomer from the mother liquor.

2. A method according to Claim 1, wherein the crystals rich in X-isomer are formed in the mixture before the removing step.

3. A method according to Claim 2, wherein the crystallization is carried out in a solvent.

4. A method according to Claim 3, wherein the solvent comprises an alcohol, an aliphatic hydrocarbon and/or an alicyclic hydrocarbon, optionally together with an aromatic hydrocarbon.

5. A method according to Claim 4, wherein the solvent comprises an alkanol having 1 to 4 carbon atoms.

6. A method according to Claim 2, 3, 4 or 5, which is carried out in the presence of crystalline X-isomer or a crystalline mixture of the ester rich in said X-isomer.

7. A method according to Claim 6, wherein a crystalline X-isomer or a crystalline mixture of the ester rich in said X-isomer is added as a seed crystal.

8. A method according to Claim 1, wherein a solidified mixture of said X-isomer and said Y-isomer is partially dissolved in a solvent and then the solid is removed to obtain an ester rich in said Y-isomer from the solution.

9. A method according to any of Claims 1 to 8, wherein the ratio of X-isomer to Y-isomer in the initial mixture is 35:65 to 65:35.

10. A method according to any of Claims 1 to 9, wherein the minimum amount of X-isomer in the X-rich crystals removed is 59% and the minimum amount of Y-isomer in the mother liquor is 59%.

## Patentansprüche

1. Ein Verfahren zur Herstellung eines Gemisches von Stereoisomeren eines $\alpha$-Cyano-3-phenoxybenzylisovaleratesterderivats der allgemeinen Formel:

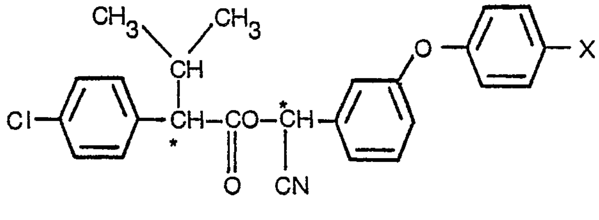

(I)

in der X ein Wasserstoff- oder Fluoratom bedeutet und * jeweils ein asymmetrisches Kohlenstoffatom anzeigt, wobei das Gemisch reich ist an "Y-Isomeren", d.h. an einem Enantiomerenpaar einer Verbindung der vorstehenden Formel mit einer (S)-Konfiguration sowohl am Säure- als auch am Alkoholrest und dessen Enantiomeren mit einer (R)-Konfiguration sowohl am Säure- als auch am Alkoholrest dadurch gekennzeichnet, daß man Kristalle einer Verbindung der vorstehenden Formel, die reich ist an "X-Isomeren", d.h. einem Enantiomerenpaar aus einer Verbindung der vorstehenden Formel mit einer (S)-Konfiguration am Säurerest und einer (R)-Konfiguration am Alkoholrest und dessen Enantiomeren mit einer (R)-Konfiguration am Säurerest und einer (S)-Konfiguration am Alkoholrest aus einem Ester der vorstehenden Formel entfernt, der ein Gemisch aus den vorgenannten X- und Y-isomeren darstellt, wobei man aus der Mutterlauge einen Ester hält, der reich ist an dem vorgenannten Y-Isomeren.

2. Verfahren nach Anspruch 1, wobei die an X-Isomeren reichen Kristalle sich im Gemisch bereits vor der Stufe des Entfernens bilden.

3. Verfahren nach Anspruch 2, wobei die Kristallisation in einem Lösungsmittel durchgeführt wird.

4. Verfahren nach Anspruch 3, wobei das Lösungsmittel einen Alkohol, einen aliphatischen Kohlenwasserstoff und/oder alicyclischen Kohlenwasserstoff, gegebenenfalls zusammen mit einem aromatischen Kohlenwasserstoff umfaßt.

5. Verfahren nach Anspruch 4, wobei das Lösungsmittel ein Alkanol mit 1 bis 4 Kohlenstoffatomen ist.

6. Verfahren nach Anspruch 2, 3, 4 oder 5, dadurch gekennzeichnet, daß es in Gegenwart von kristallinem X-Isomer oder einem kristallinen Gemisch des Esters durchgeführt wird, der reich an dem vorgenannten X-Isomeren ist.

7. Verfahren nach Anspruch 6, wobei ein kristallines X-Isomer oder ein kristallines Gemisch des Esters, der reich an vorgenanntem X-Isomeren ist, als Impfkristall zugegeben wird.

8. Verfahren nach Anspruch 1, wobei ein verfestigtes Gemisch des vorgenannten X-Isomeren und des vorgenannten Y-Isomeren teilweise in einem Lösungsmittel gelöst wird und anschließend der Feststoff entfernt wird, wobei aus der Lösung ein Ester erhalten wird, der reich ist an vorgenanntem Y-Isomeren.

9. Verfahren nach einem der Ansprüche 1 bis 8, wobei das Verhältnis von X-Isomeren zu Y-Isomeren in dem anfänglichen Gemisch 35 : 65 bis 65 : 35 beträgt.

10. Verfahren nach einem der Ansprüche 1 bis 9, wobei die Mindestmenge des X-Isomeren in den entfernten X-reichen Kristallen 59% und die Mindestmenge des Y-Isomeren in der Mutterlauge 59% beträgt.

**Revendications**

1. Un procédé de préparation d'un mélange de stéréoisomères d'un dérivé de l'ester isovalérate d'$\alpha$-cyano-3-phénoxybenzyle répondant à la formule générale:

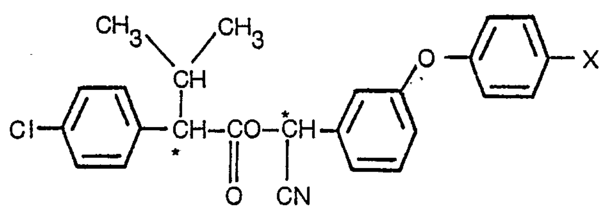

dans laquelle X est un atome d'hydrogène ou un atome de fluor, et chaque * indique un atome de carbone asymétrique, ce mélange étant riche en un "isomère Y" qui est une paire d'énantiomères d'un composé répondant à ladite formule et ayant une configuration (S) sur le fragment acide et le fragment alcool et d'un énantiomère correspondant ayant une configuration (R) sur le fragment acide et le fragment alcool, lequel procédé comprend l'élimination des cristaux d'un composé répondant à ladite formule riche en un "isomère X", qui est une paire d'énantiomères constituée d'un composé répondant à ladite formule ayant une configuration (S) sur le fragment acide et une configuration (R) sur le fragment alcool et d'un énantiomère correspondant ayant une configuration (R) sur le fragment acide et une configuration (S) sur le fragment alcool, à partir d'un ester de ladite formule qui est un mélange dudit isomère X et dudit isomère Y, pour obtenir un ester riche en ledit isomère Y à partir de la liqueur mère.

2. Un procédé selon la revendication 1, dans lequel on forme les cristaux riches en isomère Y dans le mélange avant le stade d'élimination.

3. Un procédé selon la revendication 2, dans lequel la cristallisation est effectuée dans un solvant.

4. Un procédé selon la revendication 3, dans lequel le solvant comprend un alcool, un hydrocarbure aliphatique et/ou un hydrocarbure alicyclique, éventuellement avec un hydrocarbure aromatique.

5. Un procédé selon la revendication 4, dans lequel le solvant comprend un alcanol ayant 1 à 4 atomes de carbone.

6. Un procédé selon les revendications 2, 3, 4 ou 5, qu'on met en pratique en présence de l'isomère X cristallin ou d'un mélange cristallin de l'ester riche en ledit isomère X.

7. Un procédé selon la revendication 6, dans lequel on ajoute comme germe cristallin un isomère X cristallin ou un mélange cristallin de l'ester riche en ledit isomère X.

8. Un procédé selon la revendication 1, dans lequel on dissout partiellement dans un solvant un mélange solidifié dudit isomère X et dudit isomère Y puis on élimine le solide pour obtenir un ester riche en ledit isomère Y à partir de la solution.

9. Un procédé selon l'une quelconque des revendications 1 à 8, dans lequel le rapport de l'isomère X à l'isomère Y dans le mélange initial est de 35/65 à 65/35.

10. Un procédé selon l'une quelconque des revendications 1 à 9, dans lequel la quantité minimale d'isomère X dans les cristaux riches en X éliminés est de 59% et la quantité minimale de l'isomère Y dans la liqueur mère est de 59%.

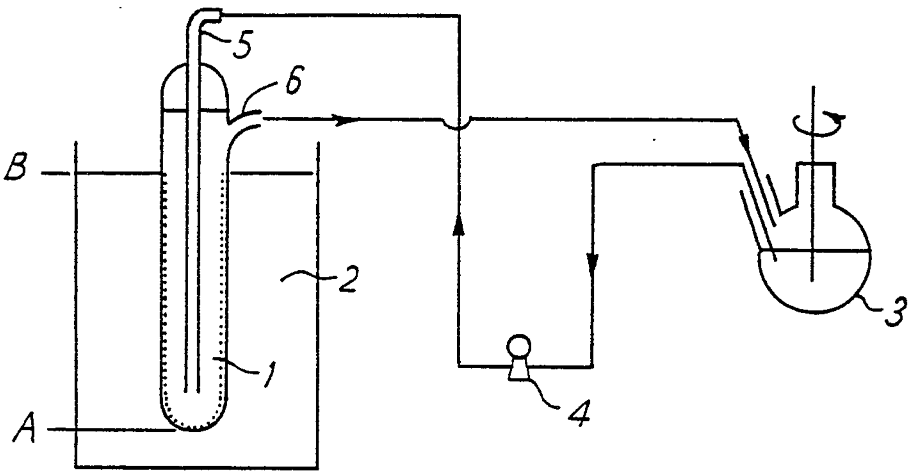
5
6
B
2
1
A
4
3